# EUROPEAN PATENT APPLICATION

(11) **EP 4 733 296 A1**
(43) Date of publication of application: **29.04.2026**
(21) Application number: 24208182.6
(22) Date of filing: 22.10.2024
(51) Int. Cl.: C07C 249/06, C07C 251/38

(54) **PROCESS FOR THE PREPARATION OF 2-C3-C4 ALKANONE OXIMES**

(71) Applicant: BASF SE, 67056 Ludwigshafen am Rhein (DE)
(72) Inventor: SCHAUB, Thomas, 67056 Ludwigshafen am Rhein (DE); ERNST, Martin, 67056 Ludwigshafen am Rhein (DE); TELES, Joaquim Henrique, 67056 Ludwigshafen am Rhein (DE); BOEHLING, Ralf, 67056 Ludwigshafen am Rhein (DE); HASHMI, A. Stephen K., 69117 Heidelberg (DE); VOLLGRAFF, Tobias, 34233 Fuldatal (DE)
(74) Representative: Meissner Bolte Partnerschaft mbB

(57) **Abstract**

The present invention provides a process for the preparation of 2-C₃-C₄ alkanone ox-imes, i.e. 2-propanoneoxime or 2-butanoneoxime, comprising the following steps:
a) subjecting a mixture (M) comprising a C₁-C₁₀ alkyl nitrite and liquefied linear C₃-C₄ alkane, i.e. n-propane or n-butane, to an irradiation by means of at least one light-emitting diode, to obtain a product mixture (P), which contains 2-C₃-C₄ alkanone oxime, i.e. 2-propanoneoxime or 2-butanoneoxime, and a C₁-C₁₀ alkanol, wherein the light emitting diodes essentially emit radiation having a wavelength in the range of 300 to 500 nm; and
b) separating the C₁-C₁₀ alkanol from the product mixture (P) to obtain the 2-C₃-C₄ alkanone oxime.

## Description

The present invention relates to a process for the preparation of 2-C₃-C₄ alkanone oximes, i.e. 2-propanoneoxime or 2-butanoneoxime, which comprises reacting a C₁-C₁₀-alkyl nitrite with liquefied linear C₃-C₄ alkanone, i.e. propane or n-butane.

### BACKGROUND OF THE INVENTION

2-C₃-C₄ alkanone oximes are valuable technical compounds that are mainly used as additives in glues and as anti-skinning agents in paints, as described by J. Bielman, "Antiskinning Agents" in "Additives for Coatings", J. H. Bielman, Ed. Wiley-VCH, 2000, Weinheim.

2-C₃-C₄ alkanone oximes are currently mainly produced by the reaction of the corresponding ketones acetone or methyl-ethyl ketone (2-butanone) with hydroxylamine or its hydrochloride (see. P.G. Kletzke, The Journal of Organic Chemistry, 1964, 29, 1363-1366; J. Bielman: "Antiskinning Agents" in "Additives for Coatings", J. H. Bielman, Ed. Wiley-VCH, 2000, Weinheim). Drawbacks of these routes are, that the required hydroxylamine is difficult to handle on a production scale, since it is explosive, corrosive and poses health and enviromental hazards.

CN102070488 discloses the preparation 2-C₃-C₄ alkanone oximes involving the ammoxidation of the corresponding ketones using ammonia and hydrogen peroxide. However, this route is not well suited for production purposes either, since in particular ammonia is poisonous as well as corrosive.

It would additionally be desirable, to replace acetone or 2-butanone as starting materials for producing 2-C₃-C₄ alkanone oximes with even simpler and less expensive ones, such as especially hydrocarbons like propane or butane, which can be directly obtained in large amounts from crude oil or natural gas.

US 20110137027 discloses the nitrosation of cycloalkanes starting from nitrosyl chloride, the reaction being initiated with a light-emitting diode (LED). US 20150175531 likewise describes the preparation of cycloalkanone oximes by reacting cyloalkane with a nitrosation agent selected from nitrosyl chloride and trichloronitromethane, wherein the reaction is initiated by LED irradiation. GB 1316194 describes the nitrosation of C₁₀-C₁₃ paraffins with nitrosyl chloride and other nitrosylhalides as nitrosation agents.

A disadvantage of the processes using nitrosyl chloride is that usually substantial amounts of unwanted chlorinated by-products are obtained, whose separation from the desired products often requires tedious procedures. In addition, corrosive hydrochloric acid is formed during the reaction, which necessitates the use of expensive reactors that are stable against hydrochloric acid. Nitrosyl chloride is also corrosive and therefore also requires special reactor materials. For these reasons, these methods are very cost-intensive.

A. Mackor et al., Recueil des Travaux Chimiques des Pays-Bas 1969, 88(10), 1249-1262, describe a process for the photonitrosation of cyclohexane by means of tert-butyl nitrite, wherein the reaction is initiated by the light of a mercury vapor lamp. The resulting cyclohexanone oxime can then be further converted to caprolactam. As a major disadvantage of this method many by-products, in particular tars, are formed, requiring complex purification schemes in order to separate the various products obtained.

WO 2017133995 describes the photonitrosation of C₄-C₁₅ cycloalkanes with C₁-C₁₀-alkylnitrites by using light emitting diodes with a wavelength of 300 to 500 nm. Advantages of this process are the use of alkylnitrites instead of nitrosylchlorides and also the use of efficient LEDs instead of mercury vapor lamps. WO 2017133995 does not use linear hydrocarbons, such as propane and butane, which not only contain CH₂ groups but also CH₃ groups, suspected of causing selectivity issues.

It is therefore an object of the present invention to provide a process for the preparation of 2-C₃-C₄ alkanone oximes, i.e. 2-propanoneoxime or 2-butanoneoxime, which is not or only to a reduced extent hampered by the disadvantages of the processes described in the prior art. These and further objectives are achieved by a process for the preparation of 2-propanoneoxime and 2-butanoneoxime defined below.

### SUMMARY OF THE INVENTION

Accordingly, the present invention provides process for the preparation of 2-C₃-C₄ alkanone oximes, i.e. 2-propanoneoxime or 2-butanoneoxime, comprising the following steps:
a) subjecting a mixture (M) comprising a C₁-C₁₀ alkyl nitrite and liquefied linear C₃-C₄ alkane, i.e. n-propane or n-butane, to an irradiation by means of at least one light-emitting diode, to obtain a product mixture (P), which contains 2-C₃-C₄ alkanone oxime, i.e. 2-propanoneoxime or 2-butanoneoxime, and a C₁-C₁₀ alkanol, wherein the light emitting diodes essentially emit radiation having a wavelength in the range of 300 to 500 nm, in particular in the range of 340 to 390 nm and especially in the range of 350 to 370 nm; and
b) separating the C₁-C₁₀ alkanol from the product mixture (P) to obtain the 2-C₃-C₄ alkanone oxime.

It has surprisingly been found that the reaction of C₃-C₄ alkanes with C₁-C₁₀ alkyl nitrites by exposure to LED radiation within the wavelength range of 300 to 500 nm, in particular in the range of 340 to 390 nm and especially in the range of 350 to 370 nm, by the process according to the present invention provides 2-propanoneoxime and 2-butanoneoxime, respectively, in good yields and in a highly selective manner.

Moreover, the process of the invention does not require aggressive chemicals, such as nitrosyl chloride, or any reactants containing chlorine. Thus, no chlorinated by-products are generated during the process, which would generally be very difficult to separate from the product mixture (P). Since aggressive chemicals are not used in the process, there are also no special requirements for the reactors, which makes the process according to the invention extremely cost-effective.

Furthermore, the C₁-C₁₀-alcohol formed during the reaction can be reused for preparing the C₁-C₁₀-alkyl nitrite.

It is furthermore advantageous that light-emitting diodes (LEDs) are used in the method according to the invention, which are particularly energy-efficient and economical. As a result, the energy consumption of the method according to the invention is significantly reduced, in particular with respect to the methods described in the prior art, in which mercury vapor lamps are used. It is also advantageous that the LEDs do not require cooling and can therefore be used flexibly on account of the simple design and enable a simple reactor design.

Thus, the process of the present invention is easy to perform, is suitable for industrial scale production and provides the desired 2-C₃-C₄ alkanone oxime in high purity in a very efficient manner.

The 2-acetone oxime obtained according to the process of the present invention can be used for production of 2-amino-2-methylpropanol via oxidation of the oxime to 2-nitropropane, condensation with formaldehyde and hydrogenation. It can also be used as such as corrosion inhibitor, reagent in organic synthesis, determination of cobalt and ketones. 2-butanoneoxime (also known as methylethyl ketone oxime) obtained by the process of the present invention, is used as anti-skinning agent in paints.

The method according to the invention is explained in more detail below.

### DETAILED DESCRIPTION OF THE INVENTION

In the context of the present invention, the terms used generically are defined as follows:

The prefix Cₓ-C_{y} denotes the number of possible carbon atoms in the particular case.

The term "C₁-C₁₀-alkyl" denotes a linear or branched alkyl radical comprising from 1 to 10 carbon atoms. such as methyl, ethyl, propyl, 1-methylethyl (isopropyl), butyl, 1-methylpropyl (sec-butyl), 2-methylpropyl (isobutyl), 1,1-dimethylethyl (tert-butyl), pentyl, 2,2-dimethylpropyl (neo-pentyl, hexyl, heptyl, octyl, nonyl and decyl.

The reactions of the invention as described hereinafter are performed in reaction vessels customary for such reactions, the reaction being carried out in a continuous, semicontinuous or batchwise manner.

Step a) of the process according to the invention for preparing 2-C₃-C₄ alkanone oximes may be regarded as a photooximation. The conversion is effected by irradiating a mixture (M) containing C₁-C₁₀ alkyl nitrite and liquefied linear C₃-C₄ alkane with the light emitted by one or more LEDs having a wavelength of in the range of 300 to 500 nm, in particular in the range of 340 to 390 nm and especially in the range of 350 to 370 nm. The thus obtained product mixture (P) contains C₁-C₁₀ alkanol and the desired C₃-C₄ alkanone oxime.

Without being bound to theory, it is believed that during the exposure of the mixture (M) to the radiation of the LED(s), the C₁-C₁₀ alkyl nitrite contained in the mixture (M) is photochemically cleaved to obtain a nitrosyl radical and a C₁-C₁₀ alkoxy radical. It is further believed that the latter radical abstracts a hydrogen radical from the linear C₃-C₄ alkane to yield a C₁-C₁₀ alcohol and a 2-C₃-C₄ alkyl radical, which undergoes addition to the nitrosyl radical resulting in the formation of 2-nitroso-C₃-C₄ alkane as an intermediate. Under the reaction conditions, this intermediate is then isomerized to the corresponding 2-C₃-C₄ alkanone oxime.

The linear C₃-C₄ alkane for the transformation in step a) of the process of the invention is selected from the group consisting of n-propane, n-butane and mixtures thereof, and is preferably n-propane or n-butane.

Preferred C₁-C₁₀ alkyl nitrites for the transformation in step a) of the process of the invention are those having one to five carbon atoms, i.e. C₁-C₅ alkyl nitrites, such as especially selected from the group consisting of methyl nitrite, ethyl nitrite, n-propyl nitrite, isopropyl nitrite, n-butyl nitrite, sec-butyl nitrite, iso-butyl nitrite, tert-butyl nitrite, n-pentyl nitrite, 2-pentyl nitrite, 3-pentyl nitrite, 2-methyl-1-butyl nitrite, tert-pentyl nitrite, iso-pentyl nitrite and 3-methyl-2-butyl nitrite (herein also neo-pentyl nitrite).

In this context, preference is given to C₁-C₁₀ alkyl nitrites, such as in particular C₁-C₅ alkyl nitrites, whose alkyl moiety does not have a CH₂ group bound to two adjacent carbon atoms. Accordingly, more preference is given here to C₁-C₁₀ alkyl nitrites selected from the group consisting of methyl nitrite, tert-butyl nitrite and neo-pentyl nitrite and particular preference is given to tert-butyl nitrile.

According to a particularly preferred group of embodiments of the invention, the mixture (M) used in step a) of the process of the invention additionally comprises a C₁-C₁₀ alkanol. Preference is given here to those C₁-C₁₀ alkanols which are described herein below as preferred. Accordingly, particularly preference is given to methanol, tert-butanol and neo-pentanol, and specifically to tert-butanol.

According to a particular subgroup of the aforementioned group of embodiments, the C₁-C₁₀ alkyl nitrite used in reaction step a) of the process of the invention is provided to the reaction as a solution in a C₁-C₁₀ alkanol. This solution of C₁-C₁₀ alkyl nitrite in a C₁-C₁₀ alkanol is preferably obtained by the reaction of the C₁-C₁₀ alkanol with nitrogen oxides and oxygen, which is described in more detail herein below. The solution may contain water. The amount of water is typically not higher than1 % by weight, based on the total weight of the solution.

In the reaction of step a) of the process of the invention, the molar ratio of the C₁-C₁₀ alkyl nitrite, especially tert-butyl nitrite, to the liquefied linear C₃-C₄ alkane, especially propane or n-butane, in the mixture (M) subjected to the irradiation is ususally at most 1:1, preferably in the range of 1:1 to 1:100, more preferably in the range of 1:2 to 1:50, particularly in the range of 1:5 to 1:30 and specifically in the range of 1:5 to 1:20.

In the reaction of step a) of the process of the invention, the concentration of the C₁-C₁₀ alkyl nitrite, especially tert-butyl nitrite, in the mixture (M) subjected to irradiation is typically in the range of 0.1 to 40 % by weight, preferably in the range of 2 to 30 % by weight and in particular in the range of 3 to 20 % by weight based on the total weight of the mixture (M).

The one or more LEDs used in step a) of the process of the invention emit light which preferably have a wavelength in the range of 340 to 390 nm and especially in the range of 350 to 370 nm.

As well-known in the art, light-emitting diodes (LEDs) usually have an emission spectrum which has an emission band with an emission maximum. Accordingly, statements to the effect that a LED emits light having a wavelength in the range of 300 to 500 nm, in particular in the range of 340 to 390 nm and especially in the range of 350 to 370 nm, mean within the context of the present invention that the maximum of the emission band is in the range of 300 to 500 nm, in particular in the range of 340 to 390 nm and especially in the range of 350 to 370 nm. Unlike conventional UV sources, the UV light emitted by LEDs usually has a narrow bandwidth, which is typically no more than 20%, in particular not more than 15% of the wavelength at the maximum of the band. and the band width of the LED used in step a) is typically at most 100 nm or lower, in particular at most 50 nm or lower. The bandwidth is understood here as the half-width of the emission band of the LED.

In addition, emission maximum of the at least one LED used in reaction step a) of the inventive process is preferably within the range of the absorption band of the n to π* transition of the C₁-C₁₀-alkyl nitrite used. As known in the art, n to π* transition here means the transition of an electron from a non-binding n orbital of the nitrite group of the C₁-C₁₀-alkyl nitrite into one anti-binding π* orbital. The absorption range of the n to π* transition is usually in the range of 300 to 500 nm, preferably in the range of 340 to 390 nm and particularly preferably in the range of 350 to 370 nm.

The reaction in step a) of the process of the present invention may be carried out without a solvent being present, but is preferably carried out in the presence of a solvent. Suitable solvents are in particular organic solvents which are selected, for example from the group consisting of benzene, alcohols and mixtures thereof. Preferred alcohols here are the C₁-C₁₀ alkanols already mentioned above as optional component of the mixture (M) used in reaction step a), in particular those mentioned as preferable, such as methanol, tert-butanol and neo-pentanol, and specifically tert-butanol.

The total amount of the solvent used in the reaction of step a) is typically in the range of 20 to 2000 g and preferably in the range of 200 to 1200 g, based on 1 mol of the C₁-C₁₀-alkyl nitrite.

The reactants contained in the mixture (M) used in step a) can in principle be contacted with one another in any desired sequence. For example, the C₁-C₁₀ alkyl nitrite, if appropriate in dissolved form, can be initially charged and then admixed with the liquefied linear C₃-C₄ alkane. Conversely, the liquefied linear C₃-C₄ alkane, if appropriate in dissolved form, can be initially charged and admixed with the C₁-C₁₀ alkyl nitrite. Alternatively, all reactants can also be added simultaneously to the reaction vessel, optionally in premixed form.

It has been found to be beneficial to initially charge the reaction vessel with a mixture of the C₁-C₁₀ alkyl nitrite and then to add the liquefied linear C₃-C₄ alkane.

In general, the reaction in step a) is performed under temperature control. The reaction is typically effected in a closed reaction vessel with stirring apparatus. The conversion of step a) is usually performed at a temperature in the range of -30 to 150°C, preferably in the range of 0 to 120°C, more preferably in the range of 0 to 80 °C and specifically in the range of 20 to 70°C. Generally, after reaching the desired reaction temperature, the reaction mixture is irradiated by means of at least one LED with a power of 0.1 to 10 W, in particular 0.5 to 5 W, per 1 mL of the reaction mixture.

The reaction in step a) of the process of the invention is generally carried out at a pressure in the range of 1 to 30 bar, preferably in the range of 1 to 20 bar and in particular in the range of 1 to 15 bar. Specifically, the pressure is set in each case so that the linear C₃-C₄ alkane, i.e. propane or n-butane, is liquefied under the chosen conditions.

In step b) of the process of the invention the product mixture (P) obtained in step a) of the inventive process is subject to a workup procedure in order to separate the C₁-C₁₀ alkanol and, if present in the product mixture (P), unreacted linear C₃-C₄ alkane from the mixture (P) to obtain the desired 2-C₃-C₄ alkanone oxime. The separation of the linear C₃-C₄ alkane and, when applicable, of the linear C₃-C₄ alkane can be achieved by procedures well known in the art, such as particularly distilliation. The separated C₁-C₁₀ alkanol can be reused, optionally after a further purificaton step, e.g. via redistillation, for the preparation of C₁-C₁₀ alkyl nitrite by reacting it with nitrogen oxides and oxygen, which is described in more detail herein below. The separated linear C₃-C₄ alkane, in turn, can be recycled, optionally after a further purification step, e.g., by redistillation, to the reaction step a) of the process of the invention. The isolated product 2-C₃-C₄ alkanone oxime may also be subjected to a further purification step, such as e.g., redistillation, if appropriate, before it is put to further use.

The process of the present invention for the preparation of 2-C₃-C₄ alkanone oximes may optionally comprise a further reaction step, herein also called step i), wherein the C₁-C₁₀ alkyl nitrite to be used in step a) of the process is prepared by reacting C₁-C₁₀ alkanol with nitrogen oxides and oxygen. Similar reactions are known in the art and are described e.g., in EP 0 057 143 and EP 0 076 217.

Preferred C₁-C₁₀ alkanols for the conversion in step i) are those having one to five carbon atoms, i.e. C₁-C₅ alkanols, such as especially selected from the group consisting of methanol, ethanol, n-propanol, isopropanol, n-butanol, sec-butanol, iso-butanol, tert-butanol, n-pentanol, 2-pentanol, 3-pentanol, 2-methyl-1-butanol, tert-pentanol, iso-pentanol and 3-methyl-2-butanol (herein also neo-pentanol). More preference is given here to C₁-C₅ alkyl nitrites selected from the group consisting of methanol, tert-butanol and neo-pentanol and particular preference is given to tert-butanol.

As apparent from the foregoing, the C₁-C₁₀ alkyl group of the C₁-C₁₀ alkanol used in step i) is identical with the C₁-C₁₀ alkyl of the C₁-C₁₀ alkyl nitrite prepared in step i). If, for example, methanol, tert-butanol or neo-pentanol is used as C₁-C₁₀-alcohol for the conversion in step i), methyl nitrite, tert-butyl nitrite or 2,2-dimethyl-1-propyl nitrite, respectively, is obtained as C₁-C₁₀-alkyl nitrite.

The nitrogen oxides for the conversion in step i) of the process of the invention are selected from the group consisting of nitrogen monoxide (NO), nitrogen dioxide (NO₂), dinitrogen trioxide (N₂O₃) and dinitrogen pentoxide (N₂O₅).

As known in the art, nitrogen dioxide, dinitrogen trioxide and dinitrogen pentoxide can be obtained by bringing nitrogen monoxide into contact with oxygen, so that nitrogen monoxide reacts at least partially with oxygen to give nitrogen dioxide, dinitrogen trioxide and/or dinitrogen pentoxide.

Therefore, according to a particular embodiment of the invention, reaction step i) is carried out by first reacting nitrogen monoxide with oxygen to allow for the formation of nitrogen dioxide, dinitrogen trioxide and/or dinitrogen pentoxide before the alkanol is added to the reaction mixture.

In the reaction of step i) of the process of the invention, the molar ratio of the nitrogen oxides to oxygen is typically in the range of 1 to 10 and preferably in the range of 2 to 10. It is further preferred that the molar ratio of the nitrogen oxides to oxygen is > 4, such as in particular in the range of 4 to 10.

In the reaction of step i) of the inventive process, the molar ratio of the C₁-C₁₀ alkanol to the oxygen is usually in the range of 1 to 10, preferably in the range of 2 to 8 and in particular in the range of 3 to 7.

Besides the C₁-C₁₀ alkanol, nitrogen oxides and oxygen, the reaction mixture used for the reaction in step i) may optionally also contain an inert gas. Suitable inert gases are well known in the art and can be selected e.g., from the group consisting of nitrogen and carbon dioxide. If an inert gas is used at all, the reaction mixture of the conversion in step i) typically contains in the range of 1 to 99% by volume, prefer in the range of 10 to 95 % by volume and in particular in the range of 30 to 90 % by volume of inert gas, in each case based on the total volume of the reaction mixture.

To carry out the reaction of step i), the reactants can in principle be contacted with one another in any order, whereby the C₁-C₁₀ alkanol is generally used in liquid form, while the nitrogen oxides and the oxygen is generally used in gaseous form. For example, the C₁-C₁₀ alkanol, if appropriate in dissolved form, can be initially charged and then be admixed with the nitrogen oxides and the oxygen. Conversely, the nitrogen oxides and the oxygen can be initially charged and admixed with the C₁-C₁₀ alkanol. Alternatively, all reactants can also be added simultaneously to the reaction vessel. In each of these variants, the nitrogen oxides and the oxygen are preferably used in the form of a mixture, which is prepared beforehand by allowing nitrogen monoxide to react at least partially with oxygen.

It has been found to be beneficial to initially charge the reaction vessel with a nitrogen monoxide and oxygen and to allow these two gases to at least partially react with each other. To the thus formed mixture of nitrogen oxides and oxygen is then added the C₁-C₁₀ alkanol.

In general, the reaction of step i) is performed under temperature control. The reaction can be effected for example in a closed batch reactor or in a flow tube reactor. The conversion of step a) is usually performed at a temperature in the range of 10 to 300°C, preferably in the range of 20 to 130°C and in aprticular in the range of 50 to 110°C.

Depending especially on the reaction temperature, a pressure of generally in the range of 1 to 50 bar, preferably in the range of 1 to 10 bar and in particular in the range of 1 to 5 bar is established during the reaction.

The work-up of the reaction mixtures obtained in the reaction of step i) and the isolation of the C₁-C₁₀ alkyl nitrite, are effected in a customary manner, and typically include a distillation step. During work-up C₁-C₁₀ alkyl nitrite is separated from the water formed during the reaction in step i) and optionally also from unreacted C₁-C₁₀ alkanol, if present. Such a separation can be accomplished by procedures known in art, such as in particular by distillation, which allows to obtain the desired C₁-C₁₀ alkyl nitrite in high purity. The separated, unreacted C₁-C₁₀ alkanol can be recycled as starting material to step i).

The present invention is explained in more detail below by examples without restricting it thereto.

The reaction vessel used in the example was a vertically mounted 20 mL round bottom glass tube equipped with a magnetic stirring bar, gas inlet, pressure and temperature control. Temperature was controlled by means of a water bath (glass beaker) and a heatable magnetic stirring equipment. The The LED was mounted at a distance of less than 3 cm from the outer wall of the glass reactor in such a way that the cone axis of its radiation cone was perpendicular to the longitudinal axis of the reactor and irradiated the liquid in the reactor.

The following LEDs were used:
1 W LEDs: Nichia SMD LED UV NVSU233A, with 1 maximum at wavelength of 365 nm or 405 nm, 1 W of light, run at 3.7 V/1 A mounted on a heat sink (diameter: 85 mm) (examples 3, 4 and 6);
18 W LEDs: EvoluChem LEDs von Hepatochem with a single emission band wavelength of 365 nm or 380 nm, respectively (examples 1, 2 and 5).

### Examples 1 to 3:

5 mmol tert-butylnitrite and 40 mmol tert-butanol were charged to a 20 mL glas reactor. The reactor was then closed, cooled with an ice bath and 50 mmol propane where condensed to the mixture. After the additon of the propane, the reactor was warmed to room temerpature (20°C) to get a pressure in the rector of 8.4 bar. By means of a water bath, the reactor was heated to 40°C and the mixture irradiated with one of the LEDs identified in Table 1 below for 18 hours. After the reaction, the pressure was released and the amount of the formed 2-propanoneoxime as well as converted tert-butylnitrite determined via gas chromatography (see Table1).

**Table 1:**

| Example | Wavelength of LED ¹⁾ | Power of LED | GC: Yield of 2-propanoneoxime according to converted tert-butylnitrite |
|---|---|---|---|
| 1 | 365 nm | 18 watt | 63 % |
| 2 | 380 nm | 18 watt | 57 % |
| 3 | 405 nm | 1 watt | 26 % |

| | | | |
|---|---|---|---|
| 1) Maximum of emission, bandwidth < 50 nm | | | |

### Examples 4 to 6:

5 mmol tert-butylnitrite and 40 mmol tert-butanol were charged to a 20 mL glas reactor. The reactor was then closed, cooled with an ice bath and 50 mmol n-butane where condensed to the mixture. After the additon of the n-butane, the reactor was warmed to room temperature (20°C) to get a pressure in the reactor of 2.1 bar. By means of a water bath, the reactor was heated to 50°C and the mixture irradiated with one of the LEDs identified in Table 2 below for 18 hours. After the reaction, the pressure was released and the amount of the formed 2-butanoneoxime as well as converted tert-butylnitrite determined via gas chromatography (see Table 2).

**Table 2:**

| Example | Wavelength of LED ¹⁾ | Power of LED | GC: Yield 2-butanoneoxime according to converted tert-butylnitrite |
|---|---|---|---|
| 4 | 405 nm | 1 watt | 55 % |
| 5 | 365 nm | 18 watt | 90 % |
| 6 | 365 nm | 1 watt | 65 % |

| | | | |
|---|---|---|---|
| 2) Maximum of emission, bandwidth < 50 nm | | | |

## Claims

1. A process for the preparation of 2-C₃-C₄ alkanone oximes comprising the following steps:
a) subjecting a mixture (M) comprising a C₁-C₁₀ alkyl nitrite and liquefied linear C₃-C₄ alkane to an irradiation by means of at least one light-emitting diode, to obtain a product mixture (P), which contains 2-C₃-C₄ alkanone oxime and a C₁-C₁₀ alkanol, wherein the light emitting diodes essentially emit radiation having a wavelength in the range of 300 to 500 nm; and
b) separating the C₁-C₁₀ alkanol from the product mixture (P) to obtain the 2-C₃-C₄ alkanone oxime.

2. The process according to claim 1, wherein the mixture (M) additionally comprises a C₁-C₁₀ alkanol.

3. The process according to claim 1 or 2, wherein the light-emitting diode in step a) emits light having a wavelength in the range of 340 to 390 nm, especially in the range of 350 to 370 nm.

4. The process according to any one of the preceding claims, where the molar ratio of the C₁-C₁₀ alkyl nitrite to the liquefied linear C₃-C₄ alkane in the mixture (M) that is subjected to the irradiation is at most 1:1, in particular in the range of 1:1 to 1:100, preferably in the range of 1:2 to 1:50.

5. The process according to any one of the preceding claims, where the C₁-C₁₀ alkyl nitrite does not have a CH₂ group bound to two adjacent carbon atoms.

6. The process according to any one of the preceding claims, where the C₁-C₁₀ alkyl nitrite is selected from the group consisting of methyl nitrite, tert-butyl nitrite and neopentyl nitrite and where the C₁-C₁₀ alkyl nitrite is especially tert-butyl nitrite.

7. The process according to any one of the preceding claims, where the C₁-C₁₀ alkanol is selected from the group consisting of methanol, tert-butanol and neopentanol and where the C₁-C₁₀ alkanol is especially tert-butanol.

8. The process according to any one of the preceding claims, where the concentration of the C₁-C₁₀ alkyl nitrite in the mixture M subjected to irradiation is in the range of 0.1 to 40 % by weight, based on the total weight of the mixture (M).

9. The process according to any one of the preceding claims, where step a) is carried out at a temperature in the range of -30 to 150°C, in particular in the range of 0 to 120°C, especially in the range of 0 to 80°C.

10. The process according to any one of the preceding claims, where step a) is carried out at a pressure in the range of 1 to 30 bar.

11. The process according to any one of the preceding claims, where the mixture is irradiated with a power of 0.1 to 10 W, in particular 0.5 to 5 W, per 1 mL of the reaction mixture.

12. The process according to any one of the preceding claims, where the C₁-C₁₀ alkyl nitrite is provided as a solution in a C₁-C₁₀ alkanol, which is obtained by reacting the C₁-C₁₀ alkanol with nitrogen oxides and oxygen.

13. The process of claim 12, where the reaction of the C₁-C₁₀ alkanol with nitrogen oxides and oxygen is carried out at a temperature in the range of 10 to 300°C.

14. The process of any one of claims 12 or 13, where the reaction of the C₁-C₁₀ alkanol with nitrogen oxides and oxygen is carried out at a pressure in the range of 1 to 50 bar.

15. Use of any of the products obtained by the process according to any of the preceding claims for the manufacture of corrosion inhibitors, anti-skinning agents or amino alcohols like 2-amino-2-methylpropanol or 2-amino-2-methyl-butanol.
